## Europäisches Patentamt
## European Patent Office
## Office européen des brevets

(11) Numéro de publication: **0 028 557**
**B1**

(12) **FASCICULE DE BREVET EUROPEEN**

(45) Date de publication du fascicule du brevet: **10.04.85**

(21) Numéro de dépôt: **80401510.5**

(22) Date de dépôt: **23.10.80**

(51) Int. Cl.⁴: **A 61 M 5/20**

(54) **Seringue d'injection.**

(30) Priorité: **06.11.79 FR 7927275**

(43) Date de publication de la demande:
**13.05.81 Bulletin 81/19**

(45) Mention de la délivrance du brevet:
**10.04.85 Bulletin 85/15**

(84) Etats contractants désignés:
**DE GB IT**

(56) Documents cités:
**FR-A-2 193 626**
**GB-A- 16 647**
**US-A-3 605 745**

(73) Titulaire: **Bourdon, Frédéric**
**51 rue Aristide Briand**
**F-92300 Levallois (FR)**

(72) Inventeur: **Bourdon, Frédéric**
**51 rue Aristide Briand**
**F-92300 Levallois (FR)**

(74) Mandataire: **Chevallier, Robert Marie Georges**
**Cabinet BOETTCHER 23, rue La Boétie**
**F-75008 Paris (FR)**

Courier Press, Leamington Spa, England.

## Description

L'invention a pour objet une seringue comprenant un vérin à fluide sous pression qui fournit la force nécessaire à l'éjection d'un liquide ou d'une pâte.

Une telle seringue convient bien quand il s'agit de faire des piqûres dans des tissus compacts où l'infiltration d'un liquide se fait difficilement comme, par exemple, dans les tissus osseux ainsi que cela se produit fréquemment en chirurgie dentaire. Toutefois, la seringue de l'invention n'est pas limitée à cette utilisation; au contraire, elle apporte par sa conception nouvelle la possibilité de l'employer de manière plus universelle. En effet, elle permet aussi l'exécution de piqûres dans les tissus mous avec la même douceur et le même contrôle précis auxquels on parvient avec les seringues purement manuelles. A l'opposé, elle peut servir aussi à l'infiltration d'un liquide dans les tissus, sans aiguille, par une simple projection sous forte pression.

Des seringues comprenant un vérin sont déjà connues; on en trouve une description dans le brevet américain n° 3.605.745 et dans le brevet français 2.193.626. Dans ces seringues, le vérin comprend un piston sur lequel agit le fluide sous pression. Afin que l'effet de la pression soit plus facilement contrôlable, le fluide est admis sur les deux côtés du piston et c'est la pression différentielle que l'on crée entre les deux côtés qui provoque le déplacement du piston dans le sens de l'injection. La pression différentielle s'établit par suite de la manoeuvre par l'utilisateur d'une vanne d'obturation ou d'ouverture d'un orifice de fuite. Cette vanne a une course faible. En outre, sa position d'ouverture est totalement indépendante de la position du piston du vérin et, par conséquent, de la quantité de liquide injectée. L'utilisateur n'est donc nullement informé ni sur la vitesse réelle de l'injection ni sur le volume injecté à un moment donné.

Le but principal de l'invention est d'apporter une seringue assistée par un vérin grâce à laquelle l'utilisateur est renseigné à tout instant sur le volure injecté et sur la vitesse d'injection. En outre, avec la seringue de l'invention, l'opérateur régle lui-même, du bébut à la fin, la vitesse à laquelle l'injection est faite; en fait l'utilisateur impose et modifie comme il le veut, à tout moment, la vitesse ou, ce qui revient au même, le débit du liquide injecté.

Dans une seringue d'injection d'un produit contenu dans une cartouche sous l'action d'un premier piston en contact direct avec ce produit, ce premier piston est poussé par la tige d'un second piston monté coulissant dans un cylindre d'un vérin faisant partie de la seringue. Ce second piston divise le cylindre en une première chambre située du côté du produit à injecter et en une seconde chambre située du côté opposé. La première chambre est mise en communication avec l'extérieur. La seconde chambre a un orifice d'alimentation pour un fluide sous pression et communique avec l'extérieur par un passage de sortie ayant au moins un orifice pouvant d'être obturé ou dégagé à volonté par l'utilisateur, de la seringue pour commander le déplacement du second piston.

Selon l'invention, cet orifice faisant partie du passage de orfie de la seconde chambre est mobile en position par rapport au cylindre en correspondance avec le déplacement du second piston.

Dans un deuxième exemple de réalisation de l'invention le passage de sortie comprend de nombreux canaux radiaux traversant la paroi latérale du cylindre le long d'une même génératrice ayant chacun un orifice de sortie tandis qu'un élément est monté sur le cylinder de façon à vecouvrir les orifices de sortie des canaux sauf l'un au moins d'entre eux communiquant avec la seconde chambre, cet élément étant mobile pour permettre de dégager et d'obturer successivement chaque orifice de sortie.

Dans un autre exemple de réalisation de l'invention, le passage de sortie s'étend dans la tige du second piston et son orifice de sortie se trouve sur un prolongement de cette tige en dehors du cylindre. De préférence, dans un autre exemple encore de réalisation de l'invention, le passage de sortie comprend un premier tronçon s'étendant entre la seconde chambre et la première chambre en traversant le second piston et/ou la tige de ce second piston, la 1ère chambre et un second tronçou qui traverse la paroi du cylindre entre la 1ère chambre et l'extérieur du cylindre. Selon une 1ère variante de réalisation, l'élément mobile traverse la paroi extrême du cylindre à l'opposé du côté de l'éjection, cette tige se terminant à l'intérieur du cylindre par une extrémité apte à obturer l'orifice d'entrée du premier tronçon s'ouvrant dans la seconde chambre. Selon une seconde variante de réalisation, l'élément mobile traverse la paroi extrême du cylindre du côté de l'éjection et s'étend en dehors du cylinder parallèlement à ce dernier et il est muni à l'intérieur du cylindre d'un organe d'obturation de l'orifice du premier tronçon à l'intérieur de la première chambre. De préférence, dans cette seconde variante de réalisation, l'orifice d'ouverture du premier tronçon du canal de communication se trouve sur une face d'une extension radiale de la tige de second piston tournée vers le second piston et l'élément mobile est muni à l'intérieur du cylindre d'une collerette montée coulissante sur la tige de second piston entre ce piston et ladite extension radiale.

Pour bien faire comprendre l'invention, on donnera maintenant une description de plusieurs exemples de réalisation. On se reportera aux dessins annexés dans lesquels:

— la figure 1 est une vue en coupe par un plan passant par l'axe longitudinal d'une seringue conforme à l'invention,
— la figure 2 est une vue analogue à la figure 1 d'une première variante de réalisation de la même seringue,

— la figure 3 est une partielle en coupe montrant une seconde variante de réalisation,

— la figure 4 est une vue en coupe semblable aux figures 1 et 2 illustrant un exemple préféré de réalisation de l'invention.

Les exemples décrits ici présentent tous la caractéristique générale de l'invention que l'on expliquera en premier lieu avant de montrer les variantes de détail susceptibles d'être adoptées pour les modalités pratiques de réalisation. Par conséquent, sur les figures on se servira des mêmes références pour désigner les pièces identiques ou équivalentes.

Une seringue d'injection comprend un premier cylindre 1 ou une cartouche rapportée 1 contenant un liquide 2 à injecter par une aiguille 3 sous l'effet d'un premier piston 4 qui est en contact direct avec le liquide 2. Cette seringue comprend aussi en arrière du cylindre 1 ou de la cartouche 1, un cylindre 5 d'un vérin contenant un second piston 6; celui-ci est monté à une extrémité d'une tige de piston 7 qui traverse la paroi extrême 8 du côté de l'aiguille 3 ou, d'une façon plus générale, du côté de l'éjection du liquide 2 hors de la cartouche 1. L'autre extrémité de la tige de piston 7 agit directement sur le premier piston 4.

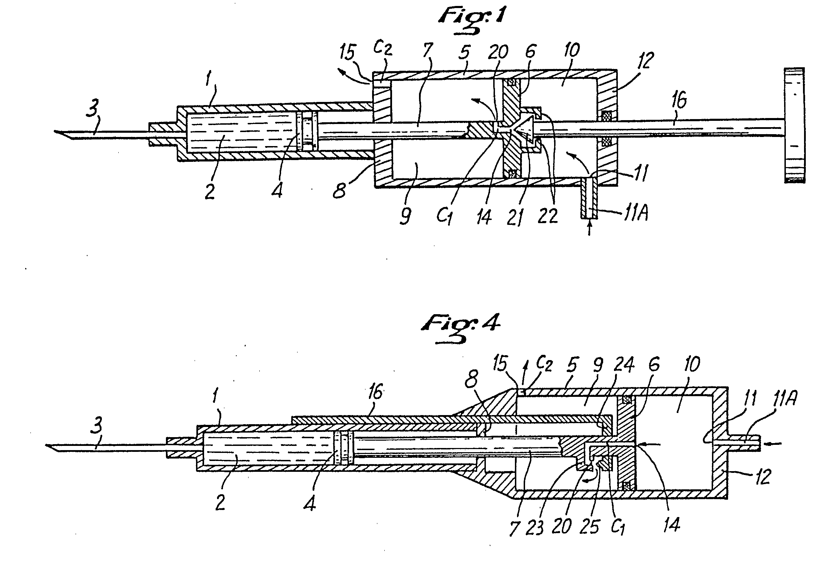

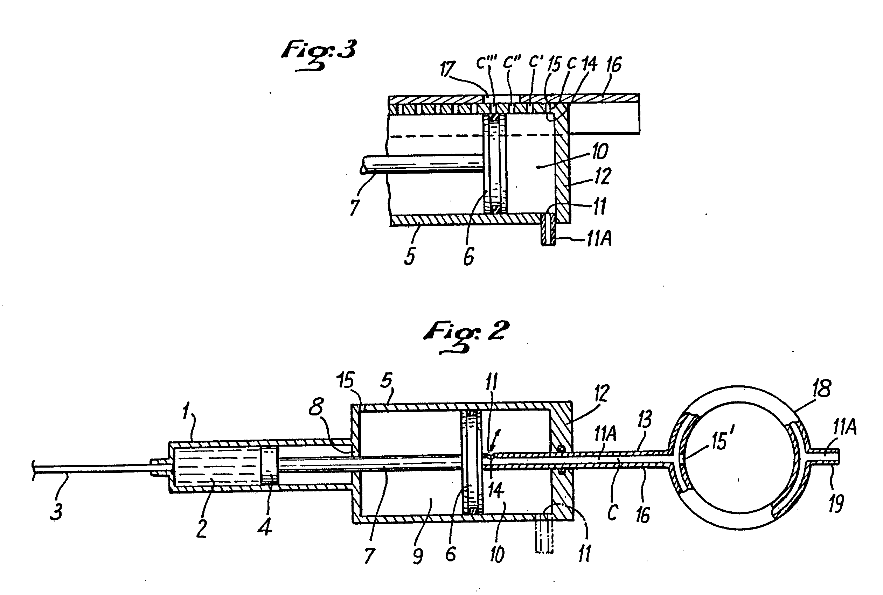

Le second piston 6 divise le cylindre 5 du vérin en une première chambre 9 située du côté du liquide 2 à éjecter et en une seconde chambre 10 située du côté opposé. Un orifice 11 d'alimentation en fluide sous pression (de préférence un gaz comprimé) s'ouvre dans la seconde chambre 10. La position exacte de cet orifice n'a pas une grande importance du moment que la seconde chambre 10 peut être alimentée convenablement. L'orifice 11 peut être celui d'un canal 11A traversant la paroi latérale du cylindre 5, comme représenté en trait plein sur la figure 1 et en trait mixte sur la figure 2, ou traversant la paroi extrême 12 du côté opposé à l'éjection (figure 4), ou l'orifice d'un canal foré dans une tige 13 fixée au piston 6 et traversant la paroi extrême 12 comme on peut le voir sur la figure 2.

Selon la caractéristique générale de l'invention, il existe au moins un canal de communication C entre la seconde chambre 10 et l'extérieur du cylindre 5; ce canal a un orifice d'entrée 14 ouvert dans la seconde chambre et un orifice de sortie 15 ou 15' ouvert à l'extérieur; en outre, l'un au moins de ces deux orifices 14, 15' est situé de façon à être obturé ou dégagé à volonté par l'utilisateur pendant le déplacement d'un élément 16 monté mobile par rapport au cylindre 5 en correspondance avec les mouvements du second piston 6.

Quand on dit que l'élément 16 est mobile en correspondance avec le piston 6, on veut dire que l'élément 16 a une course égale à celle du piston 6 et, aussi, que tout mouvement du piston 6 dans ses déplacements, en particulier dans le sens de l'injection, accompagne un mouvement correspondant de l'élément mobile 16. De préférence, pendant la course d'injection du piston 6, le déplacement correspondant de l'élément mobile 16 a lieu dans le sens longitudinal par rapport au cylindre 5.

Le canal de communication désigne, dans l'esprit de l'invention, tout passage, à voie unique ou à voies multiples, en un seul tronçon ou en plusieurs tronçons, qui peut exister entre la seconde chambre 10 et l'extérieur du cylindre 5.

Dans la seringue de la figure 3, il existe de nombreux canaux de communication C, C', C'', C''', etc... percés à travers la paroi latérale du cylindre 5, le long d'une même génératrice; ils sont espacés entre eux de distances égales ou variables dont la valeur dépend de l'importance des mouvements successifs que l'on veut imposer au second piston 6. L'élément mobile 16 est monté coulissant le long du cylindre 5 de façon à recouvrir les canaux C, C', C'', C''', etc...

Plus exactement, chaque canal, C, C', C'', C''', et... a un orifice d'entrée 14 et un orifice de sortie 15, et l'élément mobile 16 est capable de dégager puis d'obturer successivement chaque orifice 15 des canaux.

Dans l'exemple de la figure 2, il exists un seul canal de communication C qui se confond avec une partie de la longueur du canal 11A. L'orifice d'entrée 14 du canal C est confondu avec l'orifice 11 d'alimentation de la seconde chambre 10. La tige 13 présente dans sa partie extrême un anneau 18, creux, qui constitue deux branches circulaires du canal 11A après un embout extrême 19 d'alimentation en gaz sous pression. L'orifice de sortie 15' du canal C est placé à l'intérieur de l'anneau 18 de manière à pouvoir être facilement obturé ou dégagé par le pouce de l'opérateur, introduit dans cet anneau 18 de la même façon que dans un anneau classique d'une seringue manuelle. La partie de la tige 13 située entre le cylindre 5' et l'anneau 18 constitue aussi l'élément mobile 16.

Dans l'exemple des figures 1 et 4, le canal de communication C est composé d'un premier tronçon C1 et d'un second tronçon C2. Le premier tronçon C1 s'étend entre la seconde chambre 10 et la première chambre 9 en traversant le piston 6 et la tige de piston 7; le second tronçon C2 traverse la paroi du cylindre 5 entre la première chambre 9 et l'extérieur.

Dans la sevingue de la figure 1, le tronçon C1 est foré coaxialement dans le piston 6 puis dans la tige de piston 7 et il se termine par une partie radiale dans cette dernière tige. Dans la seringue de la figure 1, le tronçon C1 s'ouvre dans la première chambre 9 par un orifice d'ouverture 20 situé sur la face latérale de la tige 7; l'élément mobile 16 traverse coaxialement la paroi extrême 12, opposée au côté de l'injection, du cylindre 5 et se termine à l'intérieur de ce dernier par une extrémité conique 21 appropriée capable d'obturer l'orifice d'entrée évasé 14 du tronçon C1. De préférence, l'extrémité conique 21 a un diamètre supérieur à celui de la tige 16 et elle est prise entre des griffes repliées 22 qui s'étendent à partir de la face voisine du piston 6. Ces griffes laissent un jeu en sens longitudinal à l'extrémité conique 21.

Dans la seringue de la figure 4, la tige de piston 7 est pourvue d'une extension radiale 23 dans laquelle s'étend la partie radiale du tronçon C1; cette extension 23 est espacée du piston 6 et le tronçon C1 se termine par un orifice d'ouverture 20 situé dans la face de l'extension 23 tournée vers le piston 6. L'élément mobile 16 traverse la paroi extrême 8, du côté de l'éjection, du cylindre 5, et se termine à l'intérieur de ce dernier par une collerette 24 montée coulissante sur la tige de piston 7 entre le piston 6 et l'extension radiale 23. La collerette 24 porte un organe d'obturation 25 capable de venir obturer l'orifice d'ouverture 20 du tronçon C1 à l'intérieur de la première chambre 9. L'élément mobile 16 traverse la paroi extrême 8 du cylindre 5, et peut glisser en sens longitudinal le long de la cartouche 1.

Le fonctionnement est identique dans tous les cas. Quand la seringue a été mise en état d'utilisation, lorsque du gaz sous pression est envoyé par le canal d'alimentation 11A dans la seconde chambre 10, ce gaz ne peut pas avoir d'effet sur le piston 6 tant que le canal C ou un des canaux C, C', C'', C''', etc... a ses deux orifices 14 et 15 ou 15' ouverts, ou tant que les tronçons C1, C2 ont leurs orifices ouverts. Dès que l'on obture l'un des orifices: l'orifice 15' avec son pouce dans la seringue de la figure 2, l'orifice 15 avec l'élément mobile 16 dans la seringue de la figure 3, l'orifice 14 du tronçon C1 à l'aide de l'élément mobile 16 ou l'orifice 20 du tronçon C1 à l'aide de l'élément mobile 16 respectivement dans les seringues des figures 1 et 4, la pression monte dans la seconde chambre 10 et le piston 6 est poussé dans le sens de l'injection. Ce mouvement du piston et, donc, l'injection du liquide, s'arrête aussitôt que l'orifice du canal de communication cesse d'être obturé, ce qui se produit très rapidement si l'élément mobile 16 n'est pas poussé par l'utilisateur de manière à accompagner le piston 6.

Avec la seringue de la figure 2, dès que l'anneau 18 entraîné par le piston 6 a avancé plus vite que le pouce qui obture l'orifice 15, l'injection s'arrête. Avec la seringue de la figure 3, si l'élément mobile 16 vient juste d'obturer l'orifice 15 du canal C', dès que le piston 6 dépasse le canal C'' découvert par la fenêtre 17, l'injection s'arrête. La même chose a lieu avec les seringues des figures 1 et 4.

Par conséquent, l'utilisateur commande lui-même la vitesse d'injection par l'intermédiaire de la vitesse à laquelle il déplace l'élément mobile 16. De plus, il constate et il sent de quelle façon lente ou rapide, le piston 6 exécute l'éjection du liquide en suivant le déplacement de l'élément mobile 16. Quand l'infiltration du liquide 2 se produit difficilement dans un tissu osseux, l'utilisateur déplace l'élément mobile 16 lentement à une vitesse que le piston 6 peut accompagner avec régularité. Dans des tissus mous, le déplacement de l'élément mobile peut être encore plus lent, afin qu'une éjection brutale soit évitée. Ceci est important quand on songe qu'une injection d'un liquide anesthésiant faite trop rapidement peut être la cause d'incidents cardiaques et que, faite trop brutalement, elle peut provoquer des déchirements tissulaires.

La longueur du déplacement de l'élément mobile 16 est la même que celle des pistons 6 et 4; elle est proportionnelle à la quantité de liquide éjecté. Par conséquent, la course que l'utilisateur fait accomplir à l'élément mobile 16 le renseigne sur la fraction correspondante de liquide éjecté. Il peut donc accélérer ou ralentir en conséquence le mouvement de l'élément mobile 16 ou même l'interrompre pour ne faire qu'une injection partielle dans certaines circonstances. Le mouvement peut être aussi lent qu'il le faut pour que le dosage soit précis; de plus, l'élément mobile 16 peut porter une graduation.

Bien entendu, comme avec toutes les seringues à injection assistées par un vérin, la force ou la fatigue de l'utilisateur n'interviennent plus pour limiter les injections, même dans les tissus résistants.

Avec une seringue conforme à l'invention, le piston se déplace à une vitesse aussi lente que l'utilisateur le veut, sur la totalité de sa course. Cela n'empêche pas, à l'inverse, la possibilité d'éjections très brutales car il est facile à l'utilisateur de pousser l'élément mobile 16 à la vitesse la plus grande que peut prendre le piston. Par conséquent des infiltrations sans aiguille sont possibles aussi avec la seringue de l'invention.

Les seringues des figures 1 à 3 sont relativement longues puisque la longueur de l'élément mobile 16 s'ajoute, au début de l'éjection, à celle des cylindres 1 et 5. La seringue de la figure 4 est plus courte; elle est préférable pour les soins dentaires. Dans ce cas, l'élément mobile 16 est déplacé vers l'aiguille, pendant les injections, à l'aide d'un doigt comme l'index.

On notera que la première chambre 9 a un volume décroissant pendant une course d'éjection; il est nécessaire qu'elle communique toujours librement avec l'atmosphère. Il n'est pas prévu, selon l'invention, que cette chambre 9 soit remplie de gaz sous pression afin d'équilibrer la pression exercée sur le piston 6 dans la seconde chambre 10. En conséquence, quand la résistance des tissus est faible, le piston 6 est poussé par le gaz sous pression quand l'orifice du canal de communication n'est encore que partiellement obturé. Une obturation totale n'est nécessaire que lorsque la résistance des tissus est forte. Il en résulte que, avec une seringue conforme à l'invention, la force mise en jeu est juste celle qui est nécessaire et la consommation de gaz sous pression est dosée économiquement en fonction des besoins.

Dans une seringue réalisée comme décrit en référence à la figure 4, le piston 6 avait un diamètre de 26 mm et l'orifice 20 un diamètre de 0,3 mm pour une pression du gaz d'alimentation de 4 bars.

La seringue de l'invention est utilisable pour l'injection de liquides d'anesthésie ainsi qu'on l'a dit plus haut mais il est clair qu'elle n'est pas limitée à cet usage, en raison précisément de son aptitude à créer, sous le contrôle précis et dosé de l'opérateur, une force importante d'éjection puisque l'énergie est fournie par une source extérieure. En particulier, les dentistes se servent maintenant pour la prise des empreintes dentaires d'une pâte visqueuse que l'on prépare au moment de l'emploi par le mélange d'un premier composant à base de silicone et d'un second composant contenant un durcisseur. Après préparation, cette pâte est introduite dans une seringue et éjectée à l'aide de celle-ci contre les dents et les gencives dont on veut relever l'empreinte. La seringue de l'invention convient parfaitement à cet usage.

Dans les exemples de réalisation décrits plus haut on s'est référé au cas d'une seringue servant uniquement à produire l'éjection d'un liquide ou d'une pâte par suite du déplacement asservi du piston dans le sens désirable. On ne sortirait pas du cadre de l'invention en prévoyant le déplacement asservi du piston dans le sens d'une aspiration si l'on voulait, par exemple, produire une succion forte ou brusque à l'extrémité d'un conduit monté sur le corps de la seringue. Il suffit dans ce cas de prévoir une simple inversion de la disposition des organes décrits, notamment le sens de l'arrivée et de la sortie du fluide sous pression et du déplacement de l'élément mobile commandant le débit de ce fluide.

**Revendications**

1. Seringue d'injection d'un produit comprenant un premier piston (4) en contact avec le produit (2) à injecter, ce premier piston (4) est poussé par la tige (7) d'un second piston (6) monté coulissant dans un cylindre (5), ce cylindre (5) est divisé par le second piston (6) en une première chambre (9) située du côté du produit (2) à injecter, et en une seconde chambre (10) située du côté opposé, cette première chambre (9) est mise en communication avec l'extérieur, cette seconde chambre (10) a un orifice (11, 11a) d'alimentation pour un fluide sous pression et communique avec l'extérieur par un passage de sortie (C) ayant au moins un orifice (14; 15'; 20) pouvant être obturé ou dégagé à volonté par l'utilisateur de la seringue pour commander le déplacement du second piston (6), caractérisée en ce que ledit orifice (14; 15'; 20) faisant partie du passage de sortie (C) de la seconde chambre (10) est mobile en position par rapport au cylindre (5) en correspondance avec le déplacement du second piston (6).

2. Seringue selon la revendication 1 caractérisée en ce que le second piston (6) est muni d'une tige creuse (13) s'étendant hors du cylindre (5), en ce que le passage de sortie (C) emprunte cette tige creuse (13) et en ce que ledit orifice (15') est situé sur cette tige creuse (13) en dehors du cylindre (5).

3. Seringue selon la revendication 1 caractérisée en ce que le passage de sortie (C) comprend un premier tronçon (C_1) s'étendant entre la seconde chambre (10) et la première chambre (9) en traversant le second piston (6) et/ou la tige (7) de ce second piston (6), la première chambre (9) et un second tronçon (C_2) terminé par un orifice (15) traversant la paroi du cylindre (5) entre la première chambre (9) et l'extérieur du cylindre (5) tandis qu'un élément mobile (16) traverse la paroi extrême (12) du cylindre (5) à l'opposé du côté de l'éjection et se termine à l'intérieur du cylindre (5) par une extrémité (21) apte à obturer ledit orifice (14) du premier tronçon (C_1) s'ouvrant dans la seconde chambre (10).

4. Seringue selon la revendication 3 caractérisée en ce que le premier tronçon (C_1) (C) est foré coaxialement dans le second piston (6) et dans la tige (7) de ce second piston (6) et s'ouvre dans la face latérale de cette tige (7) à l'intérieur de la première chambre (9), l'élément mobile (16) traversant coaxialement la paroi extrême (12) du cylindre (5).

5. Seringue selon la revendication 1 caractérisée en ce que le passage de sortie (C) comprend un premier tronçon (C_1) s'étendant entre la seconde chambre (10) et la première chambre (9) en traversant le second piston (6) et/ou la tige (7) de ce second piston (6), la première chambre (9) et un second tronçon (C_2) terminé par un orifice (15) traversant la paroi du cylindre (5) entre la première chambre (9) et l'extérieur du cylindre (5) tandis qu'un élément mobile (16) traverse la paroi extrême (8) du cylindre (5) du côté de l'éjection et s'étend en dehors du cylindre (5) parallèlement à ce dernier, en étant muni à l'intérieur du cylindre (5) d'un organe d'obturation (25) de ledit orifice (20) du premier tronçon (C_1) à l'intérieur de la première chambre (9).

6. Seringue selon la revendication 5 caractérisée en ce que le premier tronçon (C_1) est foré coaxialement dans le second piston (6) et dans la tige (7) de ce second piston (6), cette tige (7) ayant une extension radiale (23) avec une face tournée vers le second piston (6) et le premier tronçon (C_1) se poursuit dans cette extension radiale (23) et se termine par ledit orifice (20) dans la face tournée vers le second piston (6), l'élément mobile (16) s'étendant en face de cet orifice (20) avec une partie saillante (25) apte à s'y appliquer pour l'obturer.

7. Seringue d'injection d'un produit comprenant un premier piston (4) en contact avec le produit (2) à injecter, ce premier piston (4) est poussé par la tige (7) d'un second piston (6) monté coulissant dans un cylindre (5), ce cylindre (5) est divisé par le second piston (6) en une première chambre (9) située du côté du produit (2) à injecter, et en une seconde chambre (10) située du côté opposé, cette première chambre (9) est mise en communication avec l'extérieur, cette seconde chambre (10) a un orifice (11, 11a) d'alimentation pour un fluide sous pression et communique avec l'extérieur par un passage de sortie (C) ayant au moins un orifice (15) pouvant être obturé ou dégagé à volonté par l'utilisateur

de la seringue pour commander le déplacement du second piston (6), caractérisée en ce que le passage de sortie (C) comprend de nombreux canaux (C, C', C'', C''',...) percés à travers la paroi latérale du cylindre (5) le long d'une même génératrice, ayant chacun un orifice de sortie (15) tandis qu'un élément (16) est monté sur le cylindre (5) de façon à recouvrir les orifices de sortie (15) des canaux (C, C', C'', C''',...) sauf l'un au moins d'entre aux communiquant avec la seconde chambre (10), cet élément (16) étant mobile pour permettre de dégager et d'obturer successivement chaque orifice de sortie (15).

**Patentansprüche**

1. Injektionsspritze mit einem ersten Kolben (4), der in Anlage mit dem zu injizierenden Stoff (2) steht, und der durch die Kolbenstange (7) eines zweiten im Zylinder (5) gleitend angeordneten Kolbens (6) angeschoben wird, wobei der Zylinder (5) durch den zweiten Kolben (6) in eine erste, dem zu injizierenden Stoff (2) zugewandte Kammer (9) und in eine zweite an der entgegengesetzten Seite angeordnete Kammer (10) unterteilt wird, die erste Kammer (9) in Verbindung mit der Umgebung steht und die zweite Kammer (10) eine Zuführoffnung (11, 11a) für ein Druckfluid aufweist und mit der Umgebung über einen Auslasskanal (C) in Verbindung steht, der mindestens eine Öffnung (14; 15', 20) aufweist, die nach Wunsch vom Anwender der Spritze zur Steuerung der Verschiebung des zweiten Kolbens (6) abgedeckt oder freigegeben werden kann, dadurch gekennzeichnet, dass die Öffnung (14; 15', 20), die einen Teil des Auslasskanals (C) der zweiten Kammer (10) darstellt, gegenüber dem Zylinder (5) im Einklang mit der Verschiebung des zweiten Kolbens (6) in ihrer Stellung beweglich ist.

2. Injektionsspritze nach Anspruch 1, dadurch gekennzeichnet, dass der zweite Kolben mit einer hohlen Stange (13) ausgestattet ist, die sich jenseits des Zylinders (5) erstreckt, dass der Auslasskanal (C) diese hohle Stange (13) umfasst und dass sich die Öffnung (15) auf dieser hohlen Stange (13) ausserhalb des Zylinders (5) befindet.

3. Injektionsspritze nach Anspruch 1, dadurch gekennzeichnet, dass der Auslasskanal (C) einen ersten Abschnitt ($C_1$) aufweist, der sich zwischen der zweiten Kammer (10) und der ersten Kammer (9) erstreckt und dabei durch den zweiten Kolben (6) und/oder die Stange (7) des zweiten Kolbens (6) hindurchtritt, dass die erste Kammer (9) und ein zweiter Abschnitt ($C_2$) in eine Öffnung (15) münden, die die Wand des Zylinders (5) zwischen der ersten Kammer (9) und der Umgebung des Zylinders (5) durchdringt, während ein bewegliches Element (16) die Stirnwand (12) des Zylinders (5), die der Abgabeseite der Spritze abgewandt ist, durchdringt und im Inneren des Zylinders (5) einen Endabschnitt (21) aufweist, der die Öffnung (14) des ersten Abschnitts ($C_1$), die in die zweite Kammer (10) mündet, absperren kann.

4. Injektionsspritze nach Anspruch 3, dadurch gekennzeichnet, dass der erste Abschnitt ($C_1$) koaxial im zweiten Kolben (6) und in der Stange (7) dieses zweiten Kolbens (6) gebohrt ist und in die Seitenwand dieser Stange (7) im Inneren der ersten Kammer (9) mündet, und das bewegliche Element (16) die Stirnwand (12) des Zylinders (5) koaxial durchdringt.

5. Injektionsspritze nach Anspruch 1, dadurch gekennzeichnet, dass der Auslasskanal (C) einen ersten Abschnitt ($C_1$) aufweist, der sich zwischen der zweiten Kammer (10) und der ersten Kammer (9) erstreckt und durch den zweiten Kolben (6) und/oder die Stange (7) des zweiten Kolbens (6) hindurchtritt, dass die erste Kammer (9) und ein zweiter Abschnitt ($C_2$) in eine Öffnung (15) münden, die durch die Wand des Zylinders (5) zwischen der ersten Kammer (9) und der Umgebung des Zylinders (5) hindurchtritt, während ein bewegliches Element (16) durch die Stirnwand (8) des Zylinders (5) an der Abgabeseite der Spritze hindurchtritt und sich parallel zum Zylinder (5) über diesen hinaus erstreckt und dabei im Inneren des Zylinders (5) mit einem Sperrelement (25) für die Öffnung (20) des ersten Abschnitts ($C_1$) im Inneren der ersten Kammer (9) in Verbindung steht.

6. Injektionsspritze nach Anspruch 5, dadurch gekennzeichnet, dass der erste Abschnitt ($C_1$) axial im zweiten Kolben (6) und in der Stange (7) dieses zweiten Kolbens (6) gebohrt ist, dass diese Stange (7) einen radialen Vorsprung (23) aufweist, dessen Seite gegen den zweiten Kolben (6) gerichtet ist und der erste Abschnitt ($C_1$) sich in diesem radialen Vorsprung (23) fortsetzt und in die Öffnung (20) mündet, die in der gegen den zweiten Kolben (6) gerichteten Seite liegt, und dass das bewegliche Element (16) sich mit einem vorspringenden Abschnitt (25) gegenüber dieser Öffnung (20) erstreckt und der vorspringende Abschnitt sich gegen die Öffnung zwecks Sperrung derselben legen kann.

7. Injektionsspritze mit einem ersten Kolben (4), der in Berührung mit dem zu injizierenden Stoff (2) steht, wobei der erste Kolben (4) durch die Stange (7) eines zweiten Kolbens (6) angeschoben wird, der gleitbar im Zylinder (5) beweglich ist, der Zylinder (5) durch den zweiten Kolben (6) in eine erste, an der Seite des zu injizierenden Stoffe (2) gelegene Kammer (9) und in eine zweite an der entgegengesetzten Seite gelegene Kammer (10) unterteilt wird, die erste Kammer (9) in Verbindung mit der Umgebung steht und die zweite Kammer (10) eine Zufuhröffnung (11, 11a) für ein Druckfluid aufweist und mit der Umgebung über einen Autrittskanal (C) in Verbindung steht, der mindestens eine Öffnung (15) aufweist, die nach Wunsch durch den Anwender der Spritze gesperrt oder freigegeben werden kann, um die Verschiebung des zweiten Kolbens (6) zu steuern, dadurch gekennzeichnet, dass der Auslasskanal (C) eine Anzahl von Kanälen (C, C', C'', C''',...) aufweist, die durch die Seitenwand des Zylinders (5) längs der gleichen Erzeugenden hindurchtreten, wovon jeder eine Austrittsöffnung (15) aufweist, während ein Element (16) derart auf dem Zylinder (5) befestigt ist, dass die Auslass-öffnungen (15) der Kanäle (C, C', C'', C''',...) ausser mindestens einer derselben, die mit der zweiten Kammer (10) in Verbindung stehen, abgedeckt werden, und dass dieses Element (16) beweglich angeordnet ist, um aufeinanderfolgend die Freigabe und Sperrung jeder Auslassöffnung (15) zu gestatten.

## Claims

1. A syringe for injecting a product, comprising a first piston (4) for contacting the product (2) to be injected, this first piston (4) being pushed by the rod (7) of a second piston (6) slidably mounted in a cylinder (5), this cylinder (5) being divided by the second piston (6) into a first chamber (9) located on the same side as the product (2) to be injected, and a second chamber (10) located on the opposite side, this first chamber (9) being adapted to communicate with the outside, this second chamber (10) having an orifice (11, 11a) for supplying a fluid under pressure and communicating with the outside by an outlet passage (C) having at least one orifice (14, 15', 20) which can be blocked or unblocked, as required, by the user of the syringe in order to cause displacement of the second piston (6), characterised in that the position of the said orifice (14, 15', 20) forming part of the outlet passage (C) of the second chamber (10) is moveable relative to the cylinder (5) in accordance with the displacement of the second piston (6).

2. A syringe according to Claim 1, characterised in that the second piston (6) is provided with a hollow rod (13) extending outside the cylinder (5), in that the outlet passage (C) runs along this hollow rod (13) and in that the said orifice (15') is situated on this hollow rod (13) outside of the cylinder (5).

3. A syringe according to Claim 1, characterised in that the outlet passage (C) comprises a first section $(C_1)$ extending between the second chamber (10) and the first chamber (9) and passing through the second piston (6) and/or the rod (7) of this second piston (6), the first chamber (9) and a second section $(C_2)$ ending in an orifice (15) passing through the wall of the cylinder (5) between the first chamber (9) and the outside of the cylinder (5), while a moveable element (16) passes through the end wall (12) of the cylinder (5) on the opposite side to the ejection side and has inside the cylinder (5) an end (21) adapted to block the said orifice (14) of the first section $(C_1)$ opening into the second chamber (10).

4. A syringe according to Claim 3, characterised in that the first section $(C_1)$ is drilled coaxially in the second piston (6) and in the rod (7) of this second piston (6) and emerges on the lateral surface of this rod (7) inside the first chamber (9), the moveable element (16) passing coaxially through the end wall (12) of the cylinder (5).

5. A syringe according to Claim 1, characterised in that the outlet passage (C) comprises a first section $(C_1)$ extending between the second chamber (10) and the first chamber (9) and passing through the second piston (6) and/or the rod (7) of this second piston (6), the first chamber (9) and a second section $(C_2)$ ending in an orifice (15) passing through the wall of the cylinder (5) between the first chamber (9) and the outside of the cylinder (5), while a moveable element (16) passes through the end wall (8) of the cylinder (5) on the ejection side and extends outside the cylinder (5) parallel to the latter, and is provided inside the cylinder (5) with an element (25) for blocking the said orifice (20) of the first section $(C_1)$ inside the first chamber (9).

6. A syringe according to Claim 5, characterised in that the first section $(C_1)$ is drilled coaxially in the second piston (6) and in the rod (7) of this second piston (6), this rod (7) having a radial extension (23) with a face directed towards the second piston (6), and the first section $(C_1)$ continues inside this radial extension (23) and ends in the said orifice (20) in the face directed towards the second piston (6), the moveable element (16) extending opposite this orifice (20) with a projecting part (25) adapted to be applied against it so as to block it.

7. A syringe for injecting a product, comprising a first piston (4) for contacting the product (2) to be injected, this first piston (4) being pushed by the rod (7) of a second piston (6) slidably mounted in a cylinder (5), this cylinder (5) being divided by the second piston (6) into a first chamber (9) located on the same side as the product (2) to be injected, and into a second chamber (10) located on the opposite side, this first chamber (9) being adapted to communicate with the outside, this second chamber (10) having an orifice (11, 11a) for supplying a fluid under pressure and communicating with the outside by an outlet passage (C) having at least one orifice (15) which can be blocked or unblocked, as required, by the user of the syringe, so as to cause the displacement of the second piston (6), characterised in that the outlet passage (C) comprises several channels (C, C', C'', C''',...) drilled through the side wall of the cylinder (5) along the same generatrix, and each having an outlet orifice (15) of the channels (C, C', C'', C''',...) except for at least one of them communicating with the second chamber (10), this element (16) being moveable so that each outlet orifice (15) can be blocked and unblocked in succession.

Fig.1

Fig.4

Fig.3

Fig.2

0 028 557